# EUROPÄISCHE PATENTANMELDUNG

(11) **EP 0 598 332 A2**
(43) Veröffentlichungstag der Anmeldung: **25.05.1994**
(21) Anmeldenummer: 93118263.8
(22) Anmeldetag: 11.11.1993
(51) Int. Cl.: C12Q 1/68

(54) **Einfaches Nukleinsäurevermehrungsverfahren**

(30) Priorität: 17.11.1992 DE 4238699
(71) Anmelder: BOEHRINGER MANNHEIM GMBH, D-68305 Mannheim (DE)
(72) Erfinder: Kleiber, Jörg, Dr., D-82377 Penzberg (DE); Kessler, Christoph, Dr., D-82057 Icking (DE); Majewski, Christoph, Dr., D-82392 Habach (DE)

(57) **Zusammenfassung**

Verfahren zur Vermehrung von Desoxyribonukleinsäuren nach dem Prinzip einer Transkriptionsamplifikation, wobei keine getrennte cDNA-Bildung durchgeführt werden muß.

## Beschreibung

Gegenstand der Erfindung sind ein Verfahren zur Vermehrung eines Abschnitts einer Desoxyribonukleinsäure, ein Verfahren zum Nachweis dieser Desoxyribonukleinsäure, sowie Reagenzien zur Durchführung dieser Verfahren.

Desoxyribonukleinsäuren kommen in Proben, wie z.B. Körperflüssigkeiten, nur in sehr geringen Mengen vor. Sie sind jedoch über die in ihnen enthaltene Sequenzinformation in vielen Fällen ein recht verläßlicher Indikator für die Anwesenheit bestimmter Organismen oder die Zustände von Organismen. Aus diesem Grund hat es sich bewährt, diese Sequenzinformation zu amplifizieren. Während in den ersten anwendbaren Amplifikationssystemen eine exponentielle Vermehrung der Sequenzinformation unter Durchführung von Temperaturzyklen vorgenommen wurde, gestatten neuere Systeme eine Reaktionsführung bei nahezu gleichbleibender Temperatur.

Ein solches System ist beispielsweise in der EP-A-0 329 822 beschrieben. Darin ist ein Verfahren zur Amplifikation von Ribonukleinsäuren erläutert, bei dem an ein Ende der zu amplifizierenden Nukleinsäure ein antisense-promoterhaltiges Oligonukleotid anhybridisiert und unter Verwendung der Nukleinsäure als Templat unter Anhängung von Desoxyribonukleotiden verlängert wird. Nach Denaturierung des RNA/DNA-Hybrids wird an das von der Promotersequenz entfernt liegende Ende der DNA ein anderes Oligonukleotid anhybridisiert und unter Bildung eines funktionellen Promoters verlängert.

Unter Kontrolle des Promoters wird anschließend der auf den Promoter folgende Teil des Doppelstrangs zu RNA transkribiert. Die RNA wird erneut zur Synthese des promoterhaltigen DNA-Doppelstrangs verwendet. Ein großer Nachteil dieses Verfahrens ist, daß im Falle von DNA als Ausgangstemplate die entsprechende Sequenz entweder chemisch/synthetisch hergestellt oder aus einem Plasmid mittels Restriktionsenzymen so herausgeschnitten wird, daß die am 5'-Ende gelegenen Nukleotide der zu amplifizierenden Nukleinsäure mit dem Promoteroligonukleotid hybridisieren. Ein weiterer Nachteil des Verfahrens ist, daß nach Bildung von cDNA denaturiert werden muß, was neben einer zeitlichen Verzögerung im Ablauf zu einer Zerstörung des zur Bildung der cDNA verwendeten Enzyms (z.B. bei Hitzedenaturierung) und dann erforderlichen Nachpipettierung oder Verdünnung der Probe (z.B. bei Reagenzzugabe) und Senkung der Sensitivität führt.

In der WO 91/02818 ist ein Verfahren beschrieben, welches gegenüber der EP-A-0 329 822 dahingehend konkretisiert ist, daß das 5'-Ende der zu amplifizierenden Nukleinsäure mit dem 3'-Ende der targetspezifischen Sequenz des Promoterprimers hybridisiert. Auch bei diesem Verfahren sind Vorbehandlungsschritte zur Erzeugung eines definierten 5'-Endes der zu amplifizierenden Nukleinsäure erforderlich. Diese Vorbehandlungsschritte erfordern einen Reinigungsschritt der zu amplifizierenden Nukleinsäuren vor der eigentlichen Amplifikation.

In WO 91/02818 und J. vir. Methods 35, 273-286 (1991), sowie Nature 350, 91, ist ferner unter den genannten Bedingungen ein Amplifikationsverfahren für RNA beschrieben, bei welchem in einem ersten Schritt cDNA zu der Analyt-RNA gebildet wird, dann die RNA mittels RNAse H abgedaut und dann die cDNA wie beim Nachweis von Analyt DNA weiterbehandelt wird. Dieser Prozess ist wegen der Substratspezifität von RNAse H jedoch auf RNA-Analyten beschränkt und viele Organismen oder Zustände, die auf charakteristischen DNA-Sequenzen beruhen, können so nicht nachgewiesen werden.

Aufgabe der vorliegenden Erfindung war es, ein Nukleinsäureamplifikationsverfahren für Desoxyribonukleinsäuren auf Transkriptionsbasis zu finden, welches die oben genannten Nachteile des Standes der Technik nicht aufweist und insbesondere einfacher ist.

Gegenstand der Erfindung ist daher ein Verfahren zur Vermehrung eines Abschnitts einer Desoxyribonukleinsäure A enthaltend die Schritte
a) Einzelsträngigmachen gegebenenfalls vorhandener doppelsträngiger Desoxyribonukleinsäure A unter Bildung der Stränge A- und A+;
b) Bildung eines zu Strang A- mindestens teilweise komplementären Stranges B+ durch Verlängerung eines ersten Primers P1+, welcher eine für eine Teilsequenz des Abschnitts spezifische und eine promoterhaltige Nukleotidsequenz enthält;
c) Bildung eines zu Strang B+ im wesentlichen komplementären Strangs B- durch Verlängerung eines zweiten Primers P2-, wobei die Stränge B- und B+ neben Sequenzinformation aus der Nukleinsäure A zusammen einen funktionellen Promoter für eine DNA-abhängige RNA-Polymerase enthalten;
d) Transkription des Nukleinsäuredoppelstranges aus B- und B+ unter Kontrolle des Promoters zu Ribonukleinsäure C+;
e) Bildung eines zu C+ im wesentlichen komplementären Stranges D'- durch Verlängerung eines Primers;
f) Bildung eines D'- im wesentlichen komplementären Stranges D+ durch Verlängerung eines Primers und Verlängerung von D'- zu D-, wobei die Stränge D- und D+ neben der Sequenzinformation der Ribonukleinsäure C+ zusammen einen funktionellen Promoter enthalten;
g) Transkription des Nukleinsäuredoppelstranges aus D- und D+ unter Kontrolle des Promoters zu Ribonukleinsäure C+, wobei eine oder mehrere der Reagentien zur Durchführung der Schritte c bis f bereits vor oder gleichzeitig mit der Bildung der Nukleinsäure B+ zu der Reaktionsmischung gegeben werden oder wobei zwischen Schritt a und b keine vollständige Trennung B+ von A- vorgenommen wird.

Ebenfalls Gegenstand der Erfindung sind Verfahren zum Nachweis von Desoxyribonukleinsäuren sowie Reagenzkits zur Durchführung der Verfahren, insbesondere zum Nachweis von Listeria monocytogenes.

Unter einer Desoxyribonukleinsäure wird eine Nukleinsäure verstanden, die hauptsächlich aus Desoxyribonukleotidbausteinen aufgebaut ist. Diese Desoxyribonukleotide können natürlichen Ursprungs sein. Sie können jedoch auch gegenüber den natürlichen Nukleotiden verändert sein, beispielsweise durch Anbringen einer chemischen Gruppe zur Markierung der Desoxyribonukleinsäure. Sofern es sich um am 3'-Ende zu verlängernde Nukleinsäuren handelt, können diese am 5'-Ende blockiert sein, z.B. als Desoxyribose eine Didesoxyribose aufweisen. Einen Abschnitt einer Desoxyribonukleinsäure im Sinne der Erfindung kann einzelsträngig oder doppelsträngig sein. Im Fall eines doppelsträngigen Abschnitts ist dieser auch an seinen Enden doppelsträngig (blunt ended).

Ein Primer ist eine Nukleinsäure, die, sofern sie an eine Desoxyribonukleinsäure anhybridisiert ist, als Startpunkt für die DNA-Polymeraseaktivität eines Enzyms wirken kann. Dazu ist das 3'-Ende des Primers, insbesondere das Nukleotid am 3'-Ende, komplementär zu der Desoxyribonukleinsäure, und hat eine freie, nicht phosphorylierte 3'-Hydroxylgruppe. Ein Primer enthält eine Desoxyribonukleotid-Sequenz, die für die Desoxyribonukleinsäure spezifisch ist und mit einem Bereich der DNA wegen ihrer Komplementarität hybridisieren kann. Diese Sequenz wird im folgenden als targetspezifische Sequenz bezeichnet. Die Stelle, an der die Primer auf der Desoxyribonukleinsäure hybridisieren sollen, kann dadurch festgelegt werden, daß die 3'-Enden der Primer in Bezug auf die Sequenz nach Hybridisierung mit den Strängen aufeinander zu zeigen. Bevorzugt liegt zwischen den 3'-Enden der Primer eine Nukleotidsequenz, die mindestens 1 Nukleotid, bevorzugt mindestens 30, besonders bevorzugt 40-1000 Nukleotide lang ist. Bevorzugt sind ferner die Sequenzen nicht zueinander komplementär oder untereinander hybridisierbar. Die targetspezifische Sequenz ist mindestens 10, bevorzugt 15-50, besonders bevorzugt 18-30 Nukleotide lang. Im übrigen gelten für Primer die Bedingungen, wie sie in US-A-4,683,202 angegeben sind. Die targetspezifischen Sequenzen der Primer können jedoch auch identisch sein oder identische Sequenzen beinhalten. Sie sollen jedoch nicht miteinander hybridisieren können.

Eine promoterhaltige Nukleotidsequenz ist eine Nukleotidsequenz, welche, wenn sie doppelsträngig vorläge, die Transkription des an diese Sequenz in 3'-Richtung anschließenden Nukleinsäurebereiches durch eine RNA-Polymerase imitieren würde. Die Sequenz ist vorzugsweise im Primer einzelsträngig. Sie ist vorzugsweise 17-100 Basen, besonders bevorzugt 17-50 Basen lang. Geeignete doppelsträngige Sequenzen, die eine RNA Polymerase binden kann, sind beispielsweise in Nucleic Acids Research 12, Seite 7035-7056 (1984) und in Proteinsequences and DNA Analysis 1, Seite 269-280 (1988), Biophysical Chemistry, Part III, S. 1183-1211, Freeman & Co., San Francisco, 1980; J. Bacteriol 170, S. 5248-5256 (1988); Biochem. J. 224, S. 799-815 (1984); Gene Acal. Tech. 6, S. 29-32 (1989), EP-A-0 292 802 und Nucleic acid probes, ed Symons (CRC Press, Boca Raton, 1989) bekannt.

Die Bedingungen, unter denen Primerverlängerungsreaktionen ablaufen, sind dem Fachmann aus Lehrbüchern, aber auch aus der US-A-4,683,202 bekannt. Es entsteht unter den Voraussetzungen ein Verlängerungsprodukt des Primers, welches als Matrize für die Verlängerung eines anderen Primers dienen kann, usw.

Mindestens teilweise komplementär sind im Sinne der Erfindung entweder vollständig zu einer anderen Nukleinsäure oder einem Teil einer anderen Nukleinsäure komplementäre oder zumindest soweit mit der anderen Nukleinsäure oder dem Teil der anderen Nukleinsäure komplementäre Sequenzen, sodaß sie mit der anderen Nukleinsäure unter den Bedingungen der Verlängerungsreaktion hybridisieren.

Ein funktioneller Promoter ist ein doppelsträngiger Nukleinsäurebereich, der durch Erkennung und Bindung von RNA-Polymerase die targetspezifische Synthese von RNA startet.

DNA-abhängige RNA-Polymerasen sind dem Fachmann bekannt. Sie arbeiten promoterabhängig. Als Beispiel können die Polymerasen aus den Phagen T7, SP6, N4 oder T3 dienen. Für das Transkriptionssystem von T7 wird auf Nucl. Acids Res. 15, 8783-8798 hingewiesen. Unter Transkription wird ein Vorgang verstanden, bei dem unter Verwendung eines promoterhaltigen DNA-Doppelstranges als Matrize eine Ribonukleinsäure gebildet wird, die zu einem 3' vom Ende der promoterhaltigen Sequenz des Primers in Transkriptionsrichtung liegenden Bereich eines Stranges des Doppelstranges komplementär ist. Bevorzugt im Sinne der Erfindung ist die gebildete Ribonukleinsäure komplementär zu dem Strang der DNA, welche dem erfindungsgemäßen Vermehrungsverfahren zugrundegelegt wird.

RNAse H ist ein Enzym, welches den RNA-Strang eines RNA/DNA-Hybrids verdaut, jedoch einzelsträngige RNA im wesentlichen unverdaut läßt. Es können auch andere Enzyme eingesetzt werden, sofern diese eine isotherme Strangtrennung katalysieren.

Eine Markierung im Sinne der vorliegenden Erfindung besteht aus einer direkt oder indirekt nachweisbaren Gruppe L. Direkt nachweisbare Gruppen sind beispielsweise radioaktive (³²P), farbige, oder fluoreszierende Gruppen oder Metallatome. Indirekt nachweisbare Gruppen sind beispielsweise immunologisch oder enzymatisch wirksame Verbindungen, wie Antikörper, Antigene, Haptene oder Enzyme oder enzymatisch aktive Teilenzyme. Diese werden in einer nachfolgenden Reaktion oder Reaktionssequenz detektiert. Besonders bevorzugt sind Haptene, da mit ihnen markierte Nukleosidtriphosphate (rNTP oder dNTP) im allgemeinen besonders gut als Substrate von (RNA- bzw. DNA-) Polymerasen einsetzbar sind und eine anschließende Reaktion mit einem markierten Antikörper gegen das Hapten oder das haptenisierte Nukleosid leicht vorgenommen werden kann. Solche Nukleosidtriphosphate sind beispielsweise Brom-Nukleosidtriphosphate oder Digoxigenin-, Digoxin- oder Fluorescein-gekoppelte Nukleosidtriphosphate. Als besonders geeignet haben sich die in EP-A-0 324 474 genannten Steroide und deren Detektion erwiesen. Für deren Inkorporation in Nukleinsäuren wird hiermit auf die EP-A-0 324 474 verwiesen.

Bei dem erfindungsgemäßen Verfahren handelt es sich um eine spezielle Ausführungsform der sogenannten Hybridisierungstests, die in ihren Grundzügen dem Fachmann auf dem Gebiet der Nukleinsäurediagnostik bekannt sind. Soweit experimentelle Details im folgenden nicht ausgeführt sind, wird dazu vollinhaltlich auf "Nucleic acid hybridisation", Herausgeber B.D. Hames und S.J. Higgins, IRL Press, 1986, insbesondere in den Kapiteln 1 (Hybridisation Strategy), 3 (Quantitative Analysis of Solution Hybridisation) und 4 (Quantitative Filter Hybridisation), Current Protocols in Molecular Biology, Edt. F.M. Ausubel et al., J. Wiley and Son, 1987, insbesondere 2.9.1. - 2.9.10 und Molecular Cloning, Edt. J. Sambrook et al., CSH, 1989, insbesondere 9.4.7. - 9.5.8. Bezug genommen. Dazu gehören insbesondere die bekannten Methoden zur Herstellung von markierten Nukleosidtriphosphaten, wie sie auch in EP-A-0 324 474 beschrieben sind, die chemische Synthese von modifizierten und unmodifizierten Oligonukleotiden, die Spaltung von Nukleinsäuren mittels Restriktionsenzymen, die Auswahl von Hybridisierungsbedingungen, durch welche eine Spezifität erreicht werden kann, die vom Ausmaß der Homologie zwischen den zu hybridisierenden Nukleinsäuren, deren GC-Gehalt und deren Länge abhängt, sowie die Bildung von Nukleinsäuren aus Nukleosidtriphosphaten mit Hilfe von Polymerasen, gegebenenfalls unter Verwendung von sogenannten Primern.

Verfahren zur Vermehrung von Nukleinsäuren haben zum Ziel, die Anzahl von Exemplaren einer Nukleinsäure zu erhöhen. Bei dem erfindungsgemäßen Verfahren handelt es sich um die Vermehrung eines Abschnitts einer Desoxyribonukleinsäure. Insbesondere liegt dieser Abschnitt nicht an einem der beiden Enden der Desoxyribonukleinsäure. Es ist jedoch selbstverständlich, daß mit dem vorliegenden Verfahren auch mehrere, voneinander getrennt oder nebeneinander liegende Abschnitte einer Desoxyribonukleinsäure vermehrt werden können. Sowie die Lage als auch die Länge des Abschnitts können in weiten Grenzen variiert werden. Bevorzugt ist der Abschnitt mehr als 25 Nukleotide, besonders bevorzugt zwischen 30 und 1000 Nukleotide lang.

Unter einem Nukleinsäurevermehrungsverfahren im Sinne der Erfindung wird auch ein Verfahren verstanden, bei dem die Sequenzinformation eines Abschnitts einer Nukleinsäure A vermehrt wird. Unter Sequenzinformation ist die Art und Reihenfolge von Nukleotidbasen am Zuckerphosphatrest der Nukleinsäure zu verstehen. Das erfindungsgemäße Verfahren ist sequenzspezifisch, d.h. es ist möglich, durch Wahl der Primersequenzen nur bestimmte Sequenzen bzw. Nukleinsäuren zu vermehren und andere Sequenzen in der Menge weitgehend unvermehrt zu lassen.

Die zu vermehrende Desoxyribonukleinsäure A kann beliebigen Ursprungs sein. Sie kann aus Organismen isoliert sein oder in Organismen vorliegen, aber auch chemisch oder enzymatisch synthetisiert sein. Als Organismen kommen z.B. Viren und Bakterien, jedoch auch tierische, pflanzliche oder menschliche Zellen in Frage. Es ist jedoch selbstverständlich, daß mit dem vorliegenden Verfahren auch mehrere, voneinander getrennt oder nebeneinander liegende Abschnitte einer Desoxyribonukleinsäure vermehrt werden können, sowie die Lage als auch die Länge des Abschnitts können in weiten Grenzen variiert werden. Bevorzugt ist der Abschnitt mehr als 25 Nukleotide, besonders bevorzugt zwischen 30 und 1000 Nukleotide lang. Die DNA kann auf verschiedene Weise vorbehandelt sein, die auch von einer eventuellen Isolierung aus den Organismen abhängt. Möglich, jedoch für die Schritte des erfindungsgemäßen Verfahrens nicht nötig, sind Vorbehandlungen durch Extraktion, Konzentrierung, Vermehrung, Restriktion oder cDNA-Bildung.

Die Desoxyribonukleinsäure A kann in einzelsträngiger Form als Strang A- oder in doppelsträngiger Form als Doppelstrang aus den Strängen A- und A+ vorliegen. Sofern sie doppelsträngig ist, muß sie in einem Schritt a einzelsträngig gemacht werden. Hierzu sind dem Fachmann eine Reihe von Denaturierungsmöglichkeiten bekannt. Bevorzugt ist die thermische Denaturierung, d.h. Erhitzung der Probe über den Schmelzpunkt der Desoxyribonukleinsäure A.

Sofern in der zur Verfügung stehenden Probe nur ein Strang der Desoxyribonukleinsäure A vorhanden ist, wird dieser zum Gegenstand des Vermehrungsverfahrens gemacht. Wenn beide Stränge des zu vermehrenden Abschnitts der Desoxyribonukleinsäure A in der Probe vorhanden sind, wird mindestens einer der beiden Stränge vermehrt. Der Strang der Desoxyribonukleinsäure A welcher letztendlich zur Vermehrung benutzt wird, wird im folgenden als A-bezeichnet. Die Feststellung, welcher der beiden Stränge einer doppelsträngigen Desoxyribonukleinsäure A als Grundlage für das erfindungsgemäße Verfahren benutzt werden soll und somit als A- bezeichnet werden soll, sollte vor Durchführung des Verfahrens getroffen und später nicht mehr geändert werden.

Die Bezeichnungen - und + werden im folgenden zur Charakterisierung von Nukleinsäuresträngen benutzt, um deren Orientierung in Bezug auf die Komplementarität festzulegen. So ist beispielsweise der Strang A-mindestens teilweise komplementär zu Strang A+. Die Zeichen - und + sollen jedoch kein Hinweis auf eine eventuelle Translation sein.

Auf molekularer Basis laufen nach Durchführung von Schritt a die Reaktionen b - g ab, wobei, wenn die Reaktionen nicht gestoppt werden, die Schritte e - g anschließend unter Verwendung der jeweils gebildeten Ribonukleinsäuren C+ nochmals ein- oder mehrmals hintereinander ablaufen. Dazu werden der Reaktionsmischung die Primer P1+, P2-, eine DNA-abhängige RNA-Polymerase, Ribonukleotide, Desoxyribonukleotide und Hilfsstoffe, mit denen die Bedingungen für die Schritte b - d oder b - g eingestellt werden, zugegeben. Wesentlich für eine Lösung der Aufgabe der Erfindung ist es, daß ein oder mehrere der Reagentien zur Durchführung der Schritte c - f bereits vor- oder gleichzeitig mit der Bildung der Nukleinsäure B+ zu der Reaktionsmischung zugegeben werden. Bei diesen Reagentien handelt es sich insbesondere um den zweiten Primer P2-, eine DNA-abhängige RNA-Polymerase und Ribonukleosidtriphosphate. Diese Reagentien wurden in den Verfahren, die aus dem Stand der Technik bekannt sind, erst nach Bildung der Nukleinsäure B+ und nach Denaturierung des Doppelstranges aus A- und B+ zugegeben.

In Reaktion b wird ein erster Primer P1+ mit dem Strang A-in einer Region PT1- hybridisiert. Der erste Primer P1+ weist dafür an seinem 3'-Ende eine Nukleotidsequenz PT1+ auf, welche zu dem Bereich PT1- des Stranges A- im wesentlichen komplementär ist und unter den eingestellten Bedingungen mit dieser Region hybridisieren kann. Insbesondere sind die Nukleotide am 3'-Ende perfekt komplementär zu den entsprechenden Nukleotiden im Bereich PT1-. Bevorzugt ist das 3'-Ende des Bereichs PT1- vom 3'-Ende des Stranges A- durch einen Nukleotidsequenzbereich X- entfernt, wobei dieser Bereich mindestens 1 bevorzugt mehr als 5 und besonders bevorzugt mehr als 30 Nukleotide lang ist und die Nukleotide des Bereichs X1- im wesentlichen nicht komplementär zu den entsprechenden Nukleotiden auf dem Primer P1+ sind. Dadurch wird gewährleistet, daß das 3'-Ende des Stranges A- nicht mit dem Primer P1+ hybridisiert.

Der Primer P1+ enthält neben der Nukleotidsequenz PT1+ in 5'-Richtung von PT1+ eine Nukleotidsequenz PP1+, die einem Strang einer Promotersequenz entspricht. Diese Sequenz wird im folgenden als promoterhaltig bezeichnet. Es hat sich erwiesen, daß für die Empfindlichkeit der Reaktion die Verwendung des Sense-Stranges der Promotersequenz bevorzugt ist. Der Primer P1+ kann außerdem weitere Nukleotidsequenzen PX1+ 5' der Sequenz PT1+ enthalten, bevorzugt zwischen der Sequenz PT1+ und der Sequenz PP1+. Die Sequenzen PX1+ sind bevorzugt nicht komplementär zu Sequenzen auf dem Strang A-. Es kann sich hier beispielsweise um Enhancer-Sequenzen handeln.

Die Verlängerung des Primers P1+ findet bevorzugt durch Anhängen von Monodesoxyribonukleotideinheiten an das 3'-Ende des Primers P1+ statt, wobei die angehängten Mononukleotide jeweils komplementär zu den entsprechenden Nukleotiden des Stranges A- sind. Der durch Anhängen von Nukleotiden gebildete neue Nukleotidbereich wird im folgenden mit T+ bezeichnet. Seine Länge hängt von der Fähigkeit des für die Verlängerung verwendeten Enzyms um die Bildung langer Nukleinsäure zu katalysieren. Von dieser Fähigkeit des Enzyms hängt unter anderem auch die Nukleotidsequenz des zweiten Primers P2- ab. Die Sequenz PT2- muß nämlich so gewählt sein, daß Sie innerhalb des Bereiches T+ mit B+ hybridisieren kann. PT2- ist ein Bereich, der Nukleotide aufweist, die zu Nukleotiden des Bereichs T+ komplementär sind.

Das Enzym zur Verlängerung der Primer ist für beide Primer eine DNA-Polymerase. Bevorzugt ist die Polymerase für beide Primer die gleiche, besonders bevorzugt ist Reverse Transkriptase. Sie akzeptiert nänlich sowohl RNA- als auch DNA-Einzelstränge als Matrize.

Primer P2- kann neben dem Bereich PT2- weitere Nukleotide oder Bereiche enthalten, vorzugsweise angehängt an das 5'-Ende von PT2-. Die Nukleotide am 3'-Ende von PT2- sind im wesentlichen komplementär, bevorzugt exakt komplementär zu den entsprechenden Nukleotiden des Bereiches PT2+ und entsprechen den korrespondierenden Nukleotiden des Stranges A-. Dabei kann das 5'-Ende von PT2- innerhalb oder außerhalb des 5'-Endes A+ liegen.

Bevorzugt wird der zweite Primer P2- vor- oder gleichzeitig mit der Bildung der Nukleinsäure B+ zu der Reaktionsmischung gegeben. Der erste Fall kann dadurch realisiert werden, daß beispielsweise P2- zusammen mit P1+ zu der Mischung, welche den Strang A- enthält, zugegeben wird. Bevorzugt findet nach Zugabe von P1+, besonders bevorzugt zwischen der Zugabe von P1+ und der Verlängerung des zweiten Primers P2- keine vollständige Trennung der Stränge B- und A+, d.h. keine Denaturierung, insbesondere keine thermische Denaturierung mehr statt.

Dennoch wird Primer P2- unter Verwendung des Stranges B+ als Matrize zu einem Strang B- verlängert. Dabei werden an das 3'-Ende von P2- Nukleotideinheiten angehängt. Dabei wird ein Strang B- gebildet, welcher die Bereiche PT1-, PX1- und PP1- enthält. Dieser Strang B- bildet zusammen mit Strang B+ eine doppelsträngige Nukleinsäure, welche einen funktionellen Promoter für eine DNA-unabhängige RNA-Polymerase sowie die Sequenzen PT1 und PT2 enthält.

Diese Nukleinsäure wird nun unter den im Stand der Technik EP-A-0 329 822 bekannten Bedingungen zu einer Ribonukleinsäure C+ transkribiert, anschließend ein zu C+ im wesentlichen komplementärer Strang D'- gebildet, dieser wieder als Matrize für die Bildung eines Stranges D verwendet, wobei sich erneut eine Nukleinsäure bildet, die, wie das Hybrid aus B- und B+, einen funktionellen Promoter sowie die Bereiche PT1 und PT2 enthält. Dabei wird aus dem intermediär gebildeten Strang D'- durch Verlängerung am 3'-Ende unter Verwendung von P1+ als Matrize ein Strang D- gebildet. Damit ergibt sich ein System, in welchem automatisch die Anzahl der in der Reaktionsfolge entstehenden Nukleinsäuren C+, D'-, D- und D+ vermehrt wird. Sofern die Freisetzung der Desoxyribonukleinsäure D'- mittels RNAse H Verdau vorgenommen wird, ergibt sich ein System, welches bei einer nahezu konstanten Temperatur arbeiten kann, d.h. ohne Temperaturzyklusführung auskommt. Bevorzugt ist der Fall, daß das Enzym zur Durchführung von Schritt e) dasselbe Enzym ist wie das Enzym des Schritts b). Besonders bevorzugt als Enzym is reverse Transkriptase.

Ein Vorteil des erfindungsgemäßen Verfahrens ist, daß nach Bereitstellung der einzelsträngigen Nukleinsäure A- keine vollständige Denaturierung von Desoxyribonukleinsäuredoppelsträngen erforderlich ist. Dies bedeutet die Einsparung von Schritten bzw. Geräten zur Temperaturerhöhung und anschließender Erniedrigung, oder, bei Denaturierung mittels Chemikalien, die Einsparung mindestens eines Reagenzzugabeschrittes. Hierbei muß bemerkt werden, daß es beim Nachweis von Nukleinsäuren wegen deren geringen anwesenden Menge in Proben geradezu wesentlich ist, daß Kontaminationen vermieden werden. Aus diesem Grunde ist es erstrebenswert, das Reaktionsgefäß möglichst selten zu öffnen. Im vorliegenden Fall können alle für die Vermehrung erforderlichen Reagentien zusammen zu der vorbereiteten Probenmischung mit der einzelsträngigen Nukleinsäure A- zugegeben werden. Danach muß das Reagenzgefäß erst zur Entnahme oder Weiterbearbeitung der entstandenen Nukleinsäureprodukte C+, D- oder D+ geöffnet werden. Ferner hat das erfindungsgemäße Verfahren den Vorteil, daß bei gemeinsamer Zugabe aller Reagentien nur sehr geringe Reagenzvolumina zugegeben werden müssen, sodaß die Probenflüssigkeit sehr viel weniger als in den Verfahren des Standes der Technik verdünnt wird. Dadurch wird das Verfahren empfindlicher als bei sequentieller Zugabe von Reagentien, bzw. zwischenzeitlicher Denaturierung.

Ein weiterer Vorteil des erfindungsgemäßen Verfahrens ist, daß die Nukleinsäuren A- ohne Vorbehandlung mittels Restriktionsenzymen verwendet werden können. Damit ist auch die Vermehrung von Abschnitten von genomischer DNA auf einfache Weise möglich. Es muß hier darauf hingewiesen werden, daß durch die Einsparung eines Restriktionsschnitts auch die danach erforderliche Reinigung der Nukleinsäuren von evtl. noch vorhandenem Restriktionsenzym entfällt.

Ein weiterer Vorteil ist, daß die Bildung von cDNA aus der Analyt-DNA nicht abgewartet werden muß. Dies bringt im Ablauf, auch bei automatisierten Systemen, erhebliche Verfahrensvorteile.

Die gleichzeitige Zugabe aller für die Vermehrung erforderlichen Reagenzien hat Vorteile für das Endvolumen des Vermehrungsansatzes (Verdünnungseffekt, geringeres Volumen möglich) sowie für die Handhabung (Pipettierfehler). Es ist ferner vorteilhaft, die Primer P1+ und P2- in gleichen Mengen zuzugeben. Dies kann mit einer einzigen Zugabe besonders einfach und reproduzierbar durchgeführt werden. Das erfindungsgemäße Verfahren ist besonders geeignet zur Vermehrung bzw. Nachweis von Teilen chromosomaler DNA.

Analog zu US-A-4,683,202 kann beim erfindungsgemäßen Verfahren die Variante mit sogenannten "nested" Primern angewandt werden. Man verwendet hierzu nach erfindungsgemäßer Vorvermehrung einen oder zwei Primer, deren targetspezifische Sequenzen innerhalb des amplifizierten Bereiches liegen. Dadurch wird die Selektivität der Amplifikation erhöht.

Gegenstand der Erfindung ist außerdem ein Verfahren zum Nachweis von Desoxyribonukleinsäuren A durch Durchführung der Schritte a bis g (gewünschtenfalls Schritte e-g mehrfach) des Verfahrens nach Anspruch 1 und Nachweis der gebildeten Nukleinsäuren C+, D'-, D- oder D+ oder Hybriden davon. Bei der Durchführung des Verfahrens nach Anspruch 1 entstehen in vermehrtem Umfang die Nukleinsäuren C+, D'-, D-, D+ und Hybride davon. Das erfindungsgemäße Verfahren zum Nachweis von Nukleinsäuren verwendet mindestens eine dieser gebildeten Nukleinsäuren zur Bestimmung der Anwesenheit oder der Menge an in einer Probe vorhandenen Desoxyribonukleinsäure A. Während die Schritte a - g, wie in Anspruch 1 angegeben, durchgeführt werden, wobei eine oder mehrere der Reagentien zur Durchführung der Schritte c - f bereits vor- oder gleichzeitig mit der Bildung der Nukleinsäure B+ zu der Reaktionsmischung gegeben werden oder zwischen Schritt a und b keine vollständige Trennung von B- von A+ vorgenommen wird, kann der Nachweis der gebildeten Nukleinsäuren C+, D'-, D-, D+ oder Hybriden davon auf bekannte Art und Weise durchgeführt werden. Zu diesen bekannten Schritten gehört beispielsweise die Hybridisierung der genannten Nukleinsäuren mit einer markierten Nukleinsäuresonde und Nachweis des gebildeten Hybrids, z.B. nach US-A-4,581,333 oder EP-A-0 079 139. Beim Nachweis von Hybriden der Nukleinsäuren C+, D- und D+ müssen diese vor Hybridisierung mit einer einzelsträngigen Sonde in Einzelstränge überführt werden.

Bei einer besonders bevorzugten Ausführungsform des erfindungsgemäßen Verfahrens wird in Schritt d durch Verwendung eines markierten Ribonukleosidtriphosphats eine Markierung in die Ribonukleinsäure C+ eingebaut. Bei der Markierung kann es sich sowohl um eine nachweisbare als auch um eine immobilisierbare Gruppe handeln. In diesem Fall kann die gebildete Nukleinsäure C+ entweder direkt oder nach Immobilisierung an eine feste Phase nachgewiesen werden.

Ebenfalls Gegenstand der Erfindung sind Reagenzkits für die oben genannten Verfahren. Diese enthalten
- eine Beschreibung eines Verfahrens nach Anspruch 1;
- einen Behälter 1 mit einem Enzymgemisch enthaltend eine RNA-Polymerase, einen Behälter 2 enthaltend zwei Primer mit unterschiedlicher Orientierung, wovon mindestens einer eine Promotersequenz enthält, und ein Enzym mit reverse Transkriptase-Aktivität, einen Behälter 3 enthaltend Desoxyribonukleotide und Ribonukleotide, sowie erforderlichenfalls Hilfsstoffe in einem oder mehreren geeigneten Behältern, wobei der Inhalt eines oder mehrerer der Behälter auch vereinigt sein kann;
- eine Verpackung.

Ein Reagenzkit zum Nachweis von Desoxyribonukleinsäuren A enthält außerdem bevorzugt einen Behälter mit einer markierten Nukleinsäuresonde S.

Weiterhin ist Gegenstand der Erfindung ein Satz von Oligonukleotiden zum Nachweis von Bakterien der Familie Listeria enthaltend mindestens zwei listeriaspezifische Primer 1 und 2, die unter Bedingungen der Hybridisierung der Oligonukleotide mit Nukleinsäuren aus Listeria nicht wesentlich mit sich selbst hybridisieren, wobei die Primer so beschaffen sind, daß das Verlängerungsprodukt des einen Primers als Templat für die Verlängerung des zweiten Primers dienen kann.

Einen solchen Satz bilden beispielsweise Oligonukleotide, die Nukleotidsequenzen enthalten, welche zu mindestens 90%, bevorzugt 95% homolog zu den listeriaspezifischen Sequenzen der SEQ ID NO 1-2 bzw. 1-3 sind und eine Länge von zwischen 10 und 30 Nukleotiden, bevorzugt 15-25 Nukleotiden, haben. Besonders bevorzugt sind die Oligonukleotide gleichlang oder länger als die listeriaspezifischen Bereiche der Oligonukleotide der SEQ ID NO 1-2 bzw. 1-3.

Der Abstand der einander zugewandten 3'-Enden der Primer in Bezug auf die Position der entsprechenden Nukleotide auf dem Listeria-Genom beträgt bevorzugt 20 bis 500, besondes bevorzugt 100 bis 350 Nukleotide. Einer oder mehr Primer (bevorzugt an ihren 5'-Enden) als auch die Sonde, können zusätzliche Nukleotide aufweisen, die nicht listeriaspezifisch sind. Solche Nukleotide können zum Nachweis der Verlängerungsprodukte dienen. Geeignet sind beispielsweise Promotersequenzen (besonders für die Primer für den Fall einer Transkriptionsvermehrung nach dem erfindungsgemäßen Verfahren) oder Nukleotidsequenzen, die mit weiteren, nicht listeriaspezifischen Nukleinsäuren zwecks Nachweis oder Immobilisierung hybridisieren können.

Die Primer sind unter den Hybridisierungsbedingungen der Primerelongation spezifisch für Listeria oder bevorzugt eine Spezies von Listeria (bevorzugt L. monocytogenes) und die Sonde ist bevorzugt spezifisch für Listeria monocytogenes mittels einer DNA-Polymerase. Die Sonde ist besonders bevorzugt immobilisierbar markiert.

Eine andere Definition der Listeria Primer ist möglich, aufgrund der Positionen der Listeria monocytogenes-DNA, an welchen die listeriaspezifischen Bereiche der Primer hybridisieren. Die folgenden Angaben beziehen sich auf die Sequenz des Plasmids pPLM 63 aus WO 90/08197.

| | Position |
|---|---|
| Primer 1 | 35 - 56 |
| Primer 2 | 196 - 216 |
| Sonde S | 162 - 180 |

Bevorzugt als Primer 1 ist P1+, als Primer 2 P2- und als Sonde NAS 4.

Der erfindungsgemäße Satz von Oligonukleotiden ermöglicht eine besonders spezifische Vermehrung/Nachweis von Listerien-DNA. Als spezifisch wird ein Verfahren, Primer oder Sonde bezeichnet, welches einen Nachweis von Listeria, insbesondere Listeria monocytogenes, ermöglicht, ohne daß Nukleinsäuren aus anderen, gelegentlich in den zu untersuchenden Proben vorkommenden Mikroorganismen nachgewiesen werden.

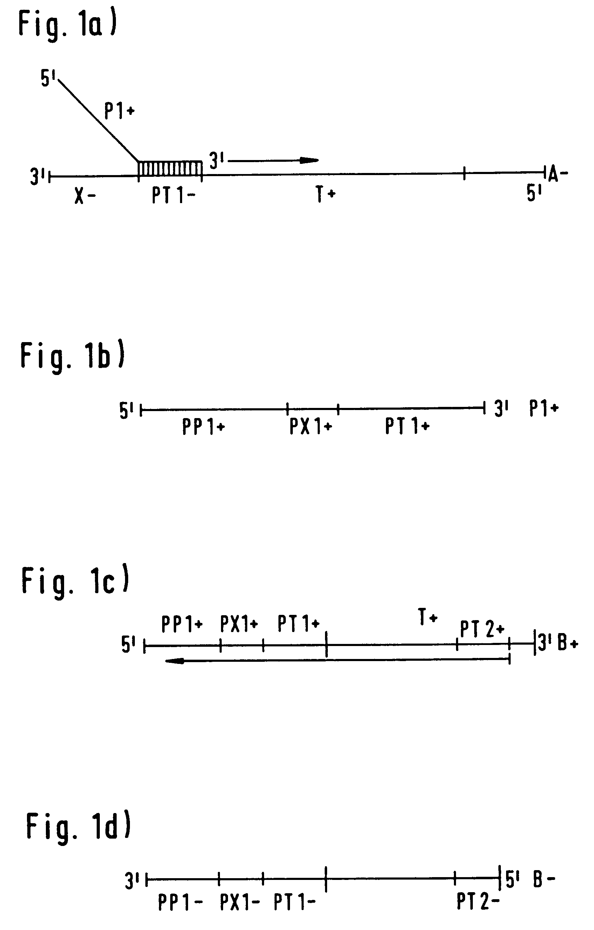

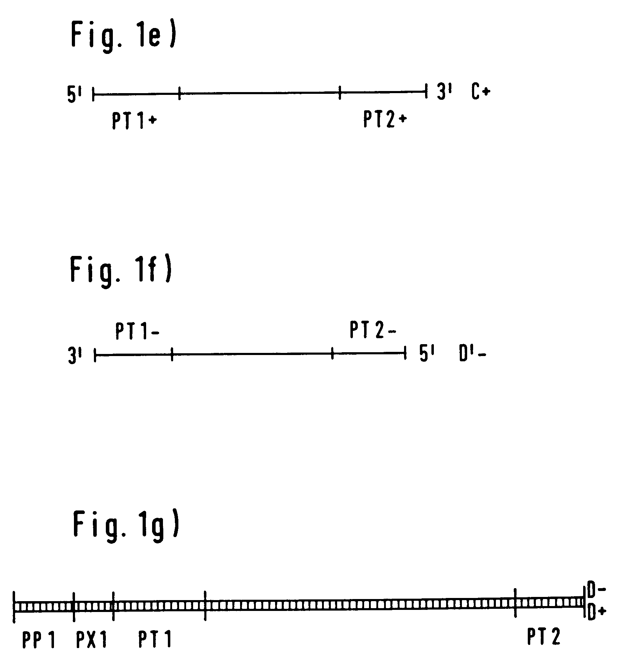

In Figur 1 ist die Bedeutung der Bezugszeichen der Beschreibung zeichnerisch dargestellt.

Figur 1a) zeigt schematisch ein Hybrid aus A- und P1+, wie es sich nach Zugabe von P1+ zu A- ausbilden muß, damit die Verlängerung von P1+ (Pfeil) stattfinden kann.

Figur 1b) zeigt schematisch den Aufbau des Primers P1+.

Figur 1c) zeigt schematisch den Aufbau von B+, sowie die Verlängerungsrichtung von P2-.

Figur 1d) zeigt schematisch den Aufbau von B-.

Figur 1e) zeigt schematisch den Aufbau von C+.

Figur 1f) zeigt schematisch den Aufbau von D'-.

Figur 1g) zeigt schematisch den Aufbau des Hybrids aus D+ und D-.

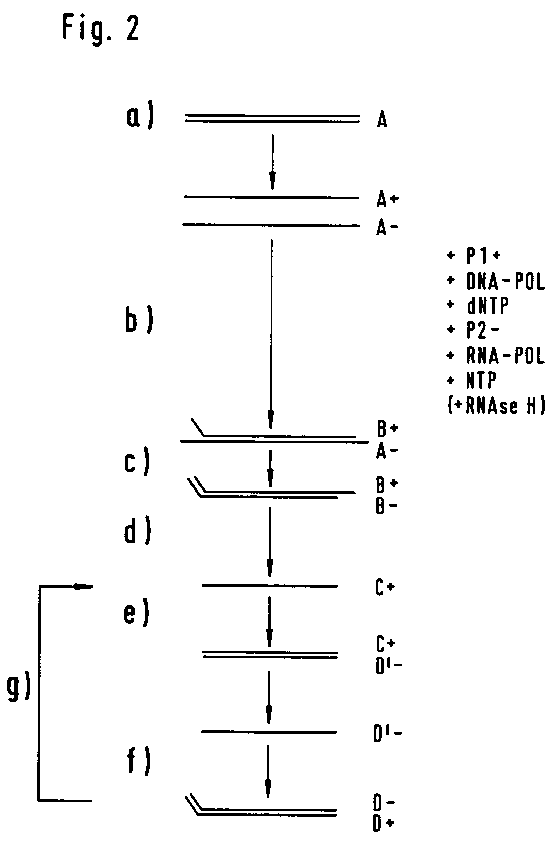

In Figur 2 ist die besonders bevorzugte Variante des erfindungsgemäßen Verfahrens schematisch wiedergegeben, bei dem alle Reagenzien zusammen nach der Denaturierung von A zugegeben werden.

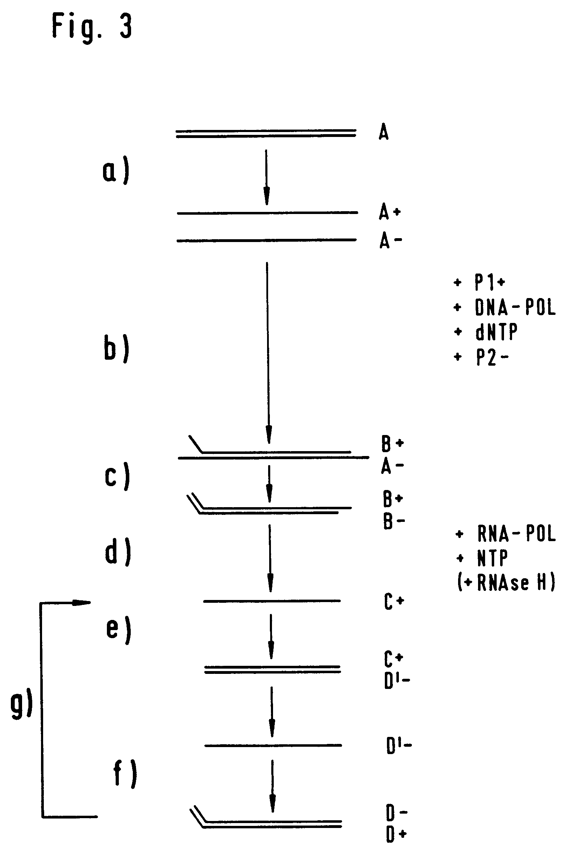

Figur 3 zeigt eine Variante des erfindungsgemäßen Verfahrens, bei dem alle Reagenzien mit Ausnahme der für die Transkription zusätzlich erforderlichen schon vor Durchführung von Schritt b) zugegeben werden.

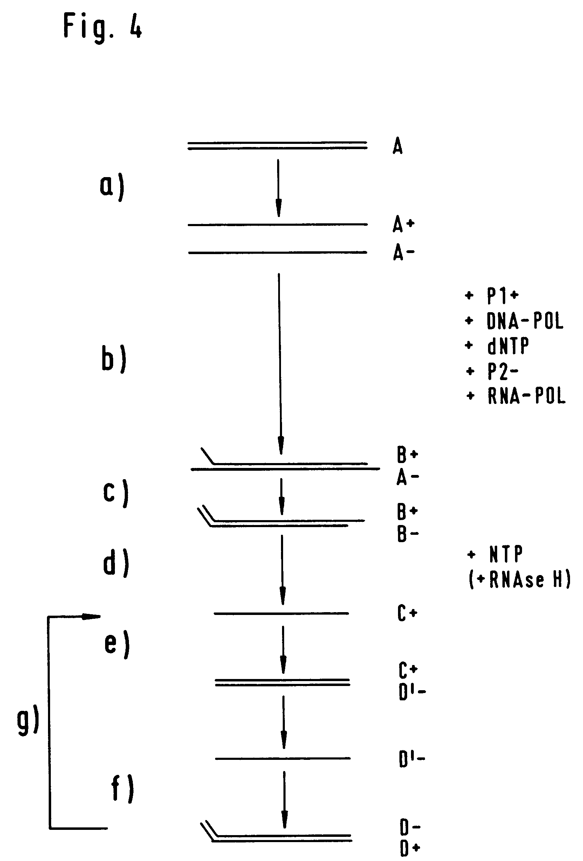

In Figur 4 ist eine Variante gezeigt, bei der nur noch NTPs und gewünschtenfalls RNAse H nach Schritt b zugegeben wurden.

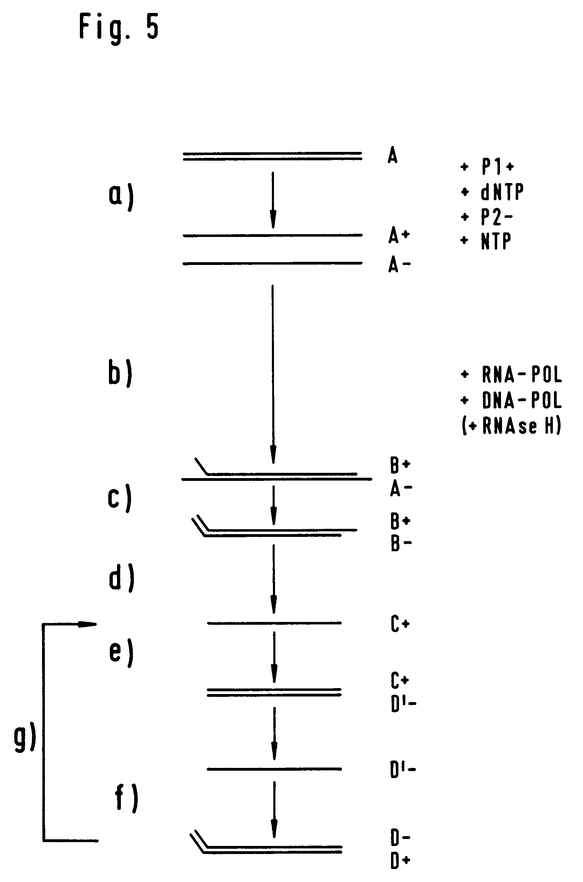

Figur 5 zeigt eine Variante, bei der P1+, P2- und Nukleosidtriphosphate schon vor oder während der Denaturierung der DNA A zugegeben werden, aber die Enzyme nach Schritt a, aber vor b zugegeben werden.

In Figur 6 ist die PIII/dth14/dth18-Region des Listeria-Monocytogenes-Chromosoms und die Lage der Primer P1+ (SEQ ID NO 1), P2- (SEQ ID NO 2) sowie einer Fangsonde S (NAS 4) (SEQ ID NO 3) (alle L. monocytogenes-spezifisch) schematisch angegeben.

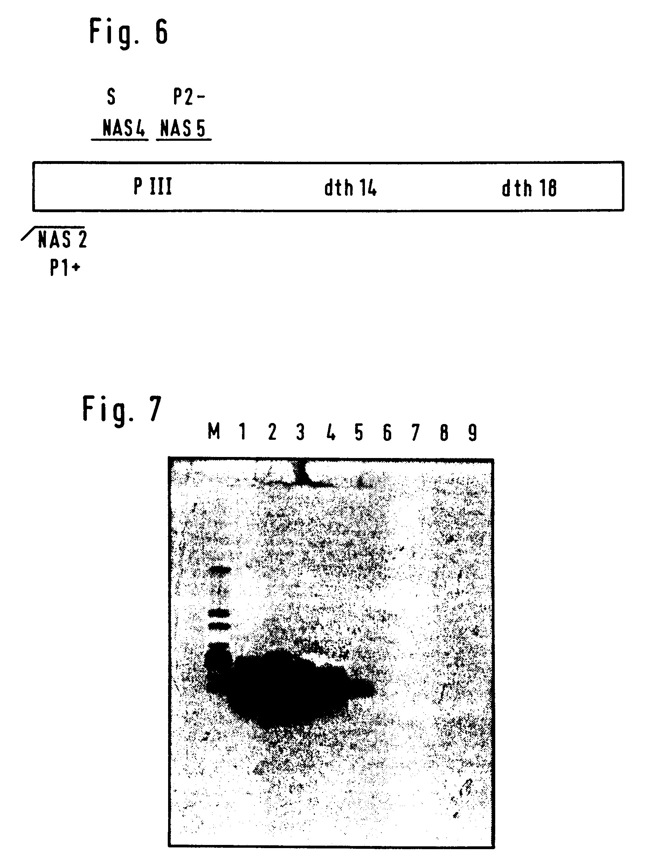

In Figur 7 ist ein Autoradiogramm von Amplifikationsprodukten gezeigt, wobei die Produkte mittels Agarosegelelektrophorese nach ihrer Größe aufgetrennt, anschließend auf eine Nylon-Membran geblottet und mit einer Digoxigenin-markierten Oligonukleotidsonde nachgewiesen wurden. Die amplifizierte Ribonukleinsäure C+ läuft als ein Produkt der Länge 181 nt. M bedeutet Molekülgewichtsmarker. In den Spuren 1-9 wurden verschiedene Mengen an chromosomaler Listeria DNA aufgegeben. (Linie 1 50 ng, Linie 2 5 ng, Linie 3 500 pg, Linie 4 50 pg, Linie 5 5pg, Linie 6 500 fg, Linie 7 50 fg, Linie 8 5 fg, Linie 9 keine chromosomale Listeria-DNA).

Die folgenden Beispiele sollen die Erfindung näher erläutern:

### Beispiel 1

### Oligonukleotide

Die Oligonukleotide der Sequenz ID's 1, 2 und 3 wurden mittels eines DNA-Synthesizers (Applied Biosystems) aus unmodifizierten bzw. mit digoxigenierten Mononukleotiden (EP-A-0 324 474) hergestellt.

### Denaturierung chromosomaler DNA

Die Denaturierungsreaktionwurde in einem Volumen von 16 µl durchgeführt. Das Reaktionsgemisch setzte sich wie folgt zusammen:
62.5 mM Tris-HCl, pH 8,5;
78.125 mM KCl;
17.75 mM MgCl₂;
1.5625 mM pro dNTP (Pharmacia);
3.125 mM pro NTP (Pharmacia) und 50 ng bis 5 fg gereinigte chromosomale DNA von Listeria monocytogenes.

Das Reaktionsgemisch wurde 15 Min. gekocht und schließlich sofort auf Eis gekühlt.

### Amplifikation denaturierter chromosomaler DNA

Die Amplifikationsreaktion wurde in einem Volumen von 25 µl in einem Eppendorf-Gefäß durchgeführt. Hierzu wurde der Denaturierungsmix (siehe oben) mit 9 µl eines zweiten Mixes, bestehend aus DTT, DMSO, Primer P1+ und P1-, RNasin, RNase H, T7 RNA-Polymerase, AMV reverse Transktriptase und BSA (alle Enzyme und BSA von Boehringer Mannheim) versetzt, sodaß die Endkonzentration des Amplifikationsmixes wie folgt war:
40 mM Tris-HCl, pH 8.5;
50 mM KCl;
12 mM Mg Cl₂;
10 mM DTT;
15% DMSO;
0,2 µM Primer P1+;
0.2 µM Primer P1-;
1 mM pro dNTP;
2 mM pro NTP;
12.5 U RNasin;
80 U T7-RNA Polymerase;
4 U AMV reverse Transkritpase;
1 U RNase H und 0,1 µg/µl BSA.

Dann wurde das Reaktionsgefäß verschlossen und bis zum Stoppen der Amplifikation nicht mehr geöffnet. Die Reaktion wurde 90 Min. bei 40° durchgeführt und schließlich durch Zugabe von 25 % Formamid und Erhitzen bei 65° für 10 Min. gestoppt.

### Nachweis der Amplifikate

a) Gelelektrophorese
   15 µl der oben genannten Reaktionsmischung, welche die amplifizierten Nukleinsäuren enthält, wurden auf ein 1,2 % Agarosegel (nach Maniatis, T., E.F.Fritsch, and J. Sambrook, 1990. Molecular Cloning: A laboratory manual. Cold Spring Harbour Laboratory, Cold Spring Harbor, New York) aufgegeben, einer Elektrophorese (100 Volt, 3h) unterworfen, anschließend auf eine Nylonmembran (Boehringer Mannheim) in einer Vakuum-Blot-Apparatur (Firma Pharmacia) geblottet.
b) Hybridisierung
   Die Membran wurde 1 h bei 45°C in 5 x SSC, 1% blocking Reagenz (Boehringer Mannheim), 0,1 % n-Laurylsarkosin (Na-Salz) und 0,02 % SDS prähybridisiert. Zur Hybridisierung wurde die Membran mit 100 ng digoxigenin-markierter Nas 4-Sonde pro ml Hybridisierungslösung (siehe oben) über Nacht bei 45°C inkubiert.
   Unspezifisch gebundene Sonde wurde wie folgt abgewaschen:
   2 x mit 2 x SSC; 0.2 % SDS bei 20°C
   2 x mit 0,1 x SSC; 0.1 % SDS bei 45°C
c) Detektion
   Die mit digoxigenin-markierter NAS 4-Sonde hybridisierten Amplifikate wurden nach Inkubation mit alkalischer Phosphatase-markierten anti-Digoxigenin Antikörpern mit AMPPD chemiluminometrisch visualisiert (für weitere Details siehe DIG Luminescent Detection Kit, Boehringer Mannheim, Best. Nr. 1363514).
   Die Membran wurde 5 Min. einem Polaroid b/w Film exponiert. Das Ergebnis zeigt Figur 7.

## Patentansprüche

1. Verfahren zur Vermehrung eines Abschnitts einer Desoxyribonukleinsäure A enthaltend die Schritte
a) Einzelsträngigmachen gegebenenfalls vorhandener doppelsträngiger Desoxyribonukleinsäure A unter Bildung der Stränge A- und A+;
b) Bildung eines zu Strang A- mindestens teilweise komplementären Stranges B+ durch Verlängerung eines ersten Primers P1+, welcher eine für eine Teilsequenz des Abschnitts spezifische und eine promoterhaltige Nukleotidsequenz enthält;
c) Bildung eines zu Strang B+ im wesentlichen komplementären Strangs B- durch Verlängerung eines zweiten Primers P2-, wobei die Stränge B- und B+ neben Sequenzinformation aus der Nukleinsäure A zusammen einen funktionellen Promoter für eine DNA-abhängige RNA-Polymerase enthalten;
d) Transkription des Nukleinsäuredoppelstranges aus B- und B+ unter Kontrolle des Promoters zu Ribonukleinsäure C+;
e) Bildung eines zu C+ im wesentlichen komplementären Stranges D'- durch Verlängerung eines Primers;
f) Bildung eines zu D'- im wesentlichen komplementären Stranges D+ durch Verlängerung eines Primers und Verlängerung von D'- zu D-, wobei die Stränge D- und D+ neben der Sequenzinformation der Ribonukleinsäure C+ zusammen einen funktionellen Promoter enthalten;
g) Transkription des Nukleinsäuredoppelstranges aus D- und D+ unter Kontrolle des Promoters zu Ribonukleinsäure C+;
dadurch gekennzeichnet, daß
eine oder mehrere der Reagentien zur Durchführung der Schritte c bis f bereits vor oder gleichzeitig mit der Bildung der Nukleinsäure B+ zu der Reaktionsmischung gegeben werden.

2. Verfahren zur Vermehrung eines Abschnitts einer Desoxyribonukleinsäure A enthaltend die Schritte
a) Einzelsträngigmachen gegebenenfalls vorhandener doppelsträngiger Desoxyribonukleinsäure A unter Bildung der Stränge A- und A+;
b) Bildung eines zu Strang A- mindestens teilweise komplementären Stranges B+ durch Verlängerung eines ersten Primers P1+, welcher eine für eine Teilsequenz des Abschnitts spezifische und eine promoterhaltige Nukleotidsequenz enthält;
c) Bildung eines zu Strang B+ im wesentlichen komplementären Strangs B- durch Verlängerung eines zweiten Primers P2-, wobei die Stränge B- und B+ neben Sequenzinformation aus der Nukleinsäure A zusammen einen funktionellen Promoter für eine DNA-abhängige RNA-Polymerase enthalten;
d) Transkription des Nukleinsäuredoppelstranges aus B- und B+ unter Kontrolle des Promoters zu Ribonukleinsäure C+;
e) Bildung eines zu C+ im wesentlichen komplementären Stranges D'- durch Verlängerung eines Primers;
f) Bildung eines D'- im wesentlichen komplementären Stranges D+ durch Verlängerung eines Primers und Verlängerung von D'- zu D-, wobei die Stränge D- und D+ neben der Sequenzinformation der Ribonukleinsäure C+ zusammen einen funktionellen Promoter enthalten;
g) Transkription des Nukleinsäuredoppelstranges aus D- und D+ unter Kontrolle des Promoters zu Ribonukleinsäure C+;
dadurch gekennzeichnet, daß
zwischen b und c keine vollständige Trennung von B- von A+ vorgenommen wird.

3. Verfahren nach Anspruch 1 oder 2, dadurch gekennzeichnet, daß es sich bei den Reagentien um mindestens ein Reagens aus der Gruppe der RNA-Polymerase, Primer P2- und Ribonukleosidtriphosphate handelt.

4. Verfahren nach Anspruch 1, dadurch gekennzeichnet, daß nach Zugabe von P1+ keine Denaturierung von Nukleinsäuren mehr stattfindet.

5. Verfahren nach Anspruch 1 oder 2, dadurch gekennzeichnet, daß die Nukleotidsequenz der Primer so gewählt wird, daß Primer P1+ so mit der Nukleinsäure A hybridisiert, daß das 5'-Ende der Targetspezifischen Sequenz mindestens ein Nukleotid entfernt vom 3'-Ende der Nukleinsäure A-entfernt ist und zwischen dem 3'- und dem 5'-Ende der Nukleinsäure A- liegt.

6. Verfahren nach Anspruch 1 oder 2, dadurch gekennzeichnet, daß P1+ an seinem 5'-Ende den sense-Strang eines Promoters enthält.

7. Verfahren nach Anspruch 1 oder 2, dadurch gekennzeichnet, daß die Nukleinsäure A keine Promotersequenzen enthält.

8. Verfahren nach Anspruch 1 oder 2, dadurch gekennzeichnet, daß die Reagentien eine RNase H enthalten.

9. Verfahren nach Anspruch 1, dadurch gekennzeichnet, daß zwischen Schritt b und Schritt c keine Denaturierung mehr stattfindet.

10. Verfahren zum Nachweis von Desoxyribonukleinsäuren A durch
- Durchführung der Schritte a-g nach Anspruch 1; und
- Nachweis der gebildeten Nukleinsäuren C+, D'-, D- oder D+ oder Hybriden davon.

11. Reagenzkit zur Vermehrung von Nukleinsäuren enthaltend
- eine Beschreibung eines Verfahrens nach Anspruch 1;
- einen Behälter 1 mit einem Enzymgemisch enthaltend eine RNA-Polymerase,
- einen Behälter 2 enthaltend zwei Primer mit unterschiedlicher Orientierung, wovon mindestens einer eine Promotersequenz enthält, und ein Enzym mit reverse Transkriptase-Aktivität,
- einen Behälter 3 enthaltend Desoxyribonukleotide und Ribonukleotide,
- eine Verpackung.

12. Reagenzkit zum Nachweis von Nukleinsäuren enthaltend
- die Behälter 1-3 des Reagenzkits nach Anspruch 11, sowie erforderlichenfalls Hilfsstoffe in einem oder mehreren geeigneten Behältern, wobei der Inhalt eines oder mehrerer der Behälter auch vereinigt sein kann;
- einen Behälter 4, enthaltend eine Nukleinsäuresonde;
- eine Beschreibung des Verfahrens nach Anspruch 10; und
- eine Verpackung.

13. Satz von Oligonukleotiden zum Nachweis von Bakterien der Familie Listeria enthaltend mindestens zwei listeriaspezifische Primer 1 und 2, die unter Bedingungen der Hybridisierung der Oligonukleotide mit Nukleinsäuren aus Listeria nicht wesentlich mit sich selbst hybridisieren, wobei die Primer so beschaffen sind, daß das Verlängerungsprodukt des einen Primers als Templat für die Verlängerung des zweiten Primers dienen kann.

14. Satz gemäß Anspruch 13, dadurch gekennzeichnet, daß er zusätzlich eine listeriaspezifische Sonde enthält, die im zwischen den Primersequenzen liegenden Bereich eines der Verlängerungsprodukte der beiden Primer hybridisieren kann.


SEQUENZPROTOKOLL

(1) ALLGEMEINE INFORMATION:

(i) ANMELDER:
(A) NAME: Boehringer Mannheim GmbH
(B) STRASSE: Sandhoferstr. 116
(C) ORT: Mannheim 31
(E) LAND: DE
(F) POSTLEITZAHL: 68298
(ii) ANMELDETITEL: Einfaches Nukleinsaeurevermehrungsverfahren

(iii) ANZAHL DER SEQUENZEN: 3

(iv) COMPUTER-LESBARE FORM:
(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.25 (EPA)

(2) INFORMATION ZU SEQ ID NO: 1:

(i) SEQUENZ CHARAKTERISTIKA:
(A) LÄNGE: 28 Basenpaare
(B) ART: Nukleinsäure
(C) STRANGFORM: Einzel
(D) TOPOLOGIE: linear
(ii) ART DES MOLEKÜLS: cDNS
(iii) HYPOTHETISCH: NEIN
(xi) SEQUENZBESCHREIBUNG: SEQ ID NO: 1:

AATTCTAATA CGACTCACTA TAGGGAGA 28

(2) INFORMATION ZU SEQ ID NO: 2:

(i) SEQUENZ CHARAKTERISTIKA:
(A) LÄNGE: 21 Basenpaare
(B) ART: Nukleinsäure
(C) STRANGFORM: Einzel
(D) TOPOLOGIE: linear
(ii) ART DES MOLEKÜLS: cDNS
(iii) HYPOTHETISCH: NEIN
(xi) SEQUENZBESCHREIBUNG: SEQ ID NO: 2:

GTAATCATCC GAAACCGCTC A 21

(2) INFORMATION ZU SEQ ID NO: 3:

(i) SEQUENZ CHARAKTERISTIKA:
(A) LÄNGE: 19 Basenpaare
(B) ART: Nukleinsäure
(C) STRANGFORM: Einzel
(D) TOPOLOGIE: linear
(ii) ART DES MOLEKÜLS: cDNS
(iii) HYPOTHETISCH: NEIN
(xi) SEQUENZBESCHREIBUNG: SEQ ID NO: 3:

CGTTTTACTT CTTGGACCG 19